# EUROPEAN PATENT APPLICATION

(11) **EP 2 269 504 A1**
(43) Date of publication of application: **05.01.2011**
(21) Application number: 08761521.7
(22) Date of filing: 22.04.2008
(51) Int. Cl.: A61B 5/04, A61B 5/0428

(54) **CIRCUIT FOR CONDITIONING SMALL ELECTRICAL SIGNALS AND METHOD FOR CONTROLLING SAID CIRCUIT**

(71) Applicant: Starlab Barcelona SL, 08035 Barcelona (ES)
(72) Inventor: RUFFINI, Giulio, E-08035 Barcelona (ES); DUNNE, Stephen, E-08035 Barcelona (ES); FARRES, Esteve, E-08035 Barcelona (ES)
(74) Representative: Torner Lasalle, Elisabet
(86) International application number: PCT/ES2008/000275
(87) International publication number: WO 2009/130338

(57) **Abstract**

The circuit comprises:

- an instrumentation amplifier (Amp1) with:
- a first input (Amp1+) connected to a first receiver electrode (E1) in contact with a first area (A) of a medium (H), and
- a second input (Amp1-),

- a voltage generating device (D) connected to a voltage supplying electrode (E3) in contact with a second area (C) of the medium (H) for applying a direct current reference electrical signal with a fixed value to it,
- compensation means, electrically insulated from the device (D), for compensating the direct current offsets of a weak electrical signal (SE1) received by the first electrode (E2), generating a reference voltage signal (Vref1), with a value which can be modified by means of a control system, and supplying it to the second input (Amp1-).

The method is intended for controlling the circuit.

## Description

### Field of the Invention

The present invention generally relate to a weak electrical signal, generally biological potential signal, conditioning circuit, and, in to a method for controlling such circuit, and particularly to a circuit and a method adapted for conditioning weak electrical signals coming from a medium, compensating possible interferences and direct current offsets experienced by such signals without inducing an additional current flow through the medium.

The invention is particularly applicable to conditioning weak biopotential electrical signals from any part of the body of a patient, such as those obtained by means of electroencephalograms (EEG), electrocardiograms (ECG), electro-oculograms (EOG) or electromyograms (EMG).

### Background of the Invention

Circuits used for measuring biopotential signals based on differential amplification and on filters are currently known. High-gain amplification is carried out by means of a differential amplifier with a high input impedance, a high common-mode rejection ratio (CMRR) and a gain which is adjustable.

An instrumentation amplifier is a closed-loop gain unit which has a differential input and a single output with respect to a reference terminal. The impedances of the two input terminals are generally balanced and have high values, typically 10⁹ Ω, or greater. The input polarization currents must also be low, normally between 1 nA and 50 nA. As occurs with operational amplifiers, the output impedance is very low, nominally a few milliohms, at low frequencies.

Instrumentation amplifiers are based on a low-pass filter, in which the bandwidth for a unitary gain for a small signal is typically between 500 kHz and 4 MHz. A gain increase reduces the bandwidth but its response is very flat in the range of biopotential signals (up to a few kHz). For this reason, active low-pass filters are required for improving the frequency range of interest.

An ideal low-pass filter would completely eliminate the signals above the cutoff frequency and would allow the signals below it (within the passband) to perfectly pass. Several undertakings are carried out in the actual filters in order to attempt to approximate the ideal case. Several types of filters are optimized in order to obtain a flat gain response within the passband, others sacrifice gain variation (ripple) in the passband in order to obtain a more sudden drop in the edge of the passband, whereas others sacrifice both, the flat response and the drop ratio, in order to favor the reliability in the pulsatile response.

After the analog signal conditioning, the signal can be digitized with an analog-to-digital converter, or ADC. The Nyquist criterion requires that the sampling frequency be at least two times the highest frequency contained in the signal, or the information on the signal will be lost. If the sampling frequency is less than twice the maximum frequency of the analog signal, a phenomenon known as aliasing or overlapping will occur. It is important to point out that if an input filter is not arranged in the input of the ideal sampler, any frequency component (either a signal or noise) falling outside the Nyquist bandwidth will be overlapped, i.e. will undergo aliasing effects. For this reason a filter is used in almost all the ADC sampling applications for eliminating these unwanted signals.

The main problem in biopotential measurements are the interferences, including the dominant noise of the line frequency of 50 or 60 Hz. In known proposals of weak electrical signals conditioning circuits, specifically biopotential signals obtained by means of a series of electrodes, the active electrodes include a follower close to each of them for intermediately storing the signal of the electrode, such that virtually all interference problems associated with the differential and high impedances of each electrode are eliminated.

Respective examples of the state of the art with regard to ECG systems with their corresponding instrumentation amplifiers AD8220 and INA326 respectively, appear on page 2 of the bulletin "Amplifier ICs Volume 6, Issue 1" of "Analog Devices" and on page 17 of the document "Information for Medical Applications" from "Real World Signal Processing" from Texas Instruments (SLYB108).

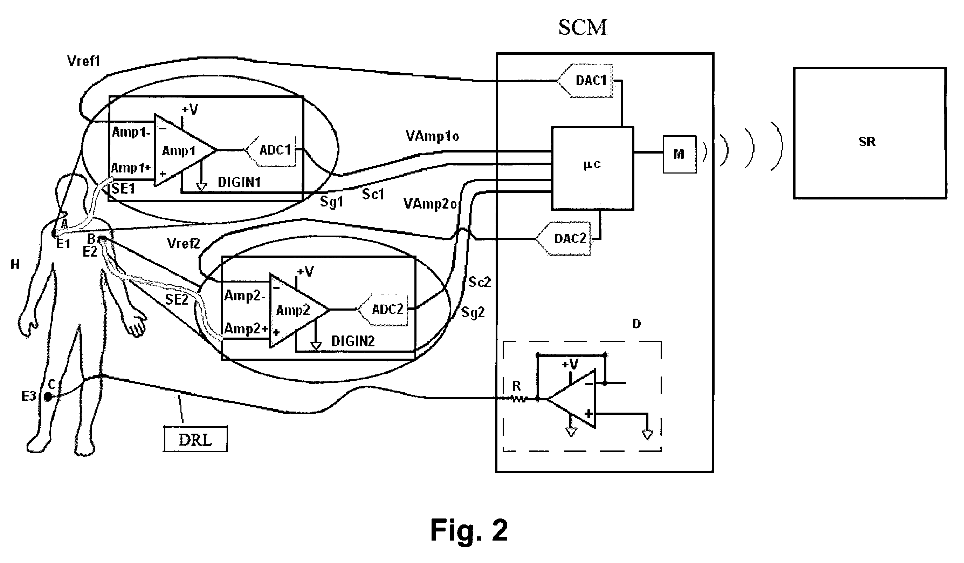

The slight imbalances in the lengths and the contacts of the electrodes cause the common-mode signal to be offset in direct current, which forms the main limitation of the differential amplifier of the instrumentation amplifier. The right leg exciter circuit (DRL) attempts to reduce this limitation by applying a voltage close to the common-mode voltage in a voltage supplying reference electrode or DRL electrode. Other alternating current (AC) coupling techniques could be applied for changing the reference voltage of the instrumentation amplifier. The circuit shown in Figure 2 of the document "Heart-Rate and EKG Monitor Using the MSP430FG439" from Texas Instruments (SLAA280) shows an example of said AC coupling techniques, in which the digital-to-analog converter DAC 1 shown therein provides a mean supply voltage level as polarization voltage for the amplifier chain.

The lower limit of the noise level in the bioelectrical measurements is determined by the thermal noise of the impedance of the electrodes. Consequently, in order to achieve noise levels in the range of microvolts, the impedance of the electrode, including that existing between the electrode and the skin of the patient, must be less than 100 kΩ.

There are several patent documents describing different systems and methods for carrying out biopotential signal measurements. Several of said documents are set forth below.

Patent document EP1631189A1 discloses a system for carrying out biopotential signal measurements, in which a probe is used with an electrode located adjacent to the patient (in contact or without contact therewith). The use of a ground voltage, the voltage of a second electrode in contact with the patient or the voltage of a second electrode incorporated in the actual probe as the reference voltage of the differential amplifier to which the electrode is connected is proposed. The probe incorporating the electrode can include electronic circuitry with amplifiers, filtering and gain steps, batteries, components for transmitting through cable or wirelessly or components for recording data for a subsequent transmission. The probe includes a conductor separated a fixed distance with respect to the electrode adjacent to the patient for the purpose of insulating it against interference signals, for example coming from the amplifier included in the actual probe.

In addition, patent US5876351A1 proposes a portable modular device for carrying out electrocardiograms, which for several of its embodiments comprises amplifiers, each of them with a first input connected to each of several electrodes, previous to the instrumentation amplifier, and with a second input connected to a common reference voltage which can be that of the DRL (Wilson) or GND electrode, selected by means of a multiplexer.

Patent US5392784A1 proposes an instrumentation amplifier applied to reducing common-mode voltage in ECG and EEG measurements by means of the application by a compensation circuit a compensation voltage representative of said common-mode voltage in different manners, some with a third DRL electrode and others without this third electrode but with a feedback path for high frequencies between a capacitor included in a compensation circuit and the capacity existing between the patient and the ground, or chassis, through said ground or chassis. The input of the amplifier of the compensation circuit is connected to the outputs of the differential amplifiers of the signals of the electrodes, for the purpose of monitoring the common-mode voltage received through the electrodes.

Patent US5713365A1 proposes dispensing with the DRL electrode by maintaining a good rejection to the electric noise, directly feeding back the common-mode voltage detected in the output of the differential amplifiers of each electrode, in the inputs thereof, for the purpose of obtaining a portable device more than that of improving the performance of the systems which do inject a reference signal through the DRL electrode.

In all the mentioned system proposals including DRL circuits there is an electrical connection between the latter and the circuit conditioning the signals coming from the electrodes, since the voltage applied to the DRL electrode corresponds to the common-mode voltage detected in the differential amplifiers connected to each electrode, which makes the fluctuations in the electrical signals provided by the electrodes and the possible artifacts, or interfering signals, cause changes in the voltage to be applied to the DRL electrode, which results in the occurrence of a current injection in the patient through the DRL electrode-skin-tissue interface, altering the balance in the potentials of the half-cells.

The differential amplifiers of each electrode in the known proposals share one and the same reference voltage.

None of the mentioned proposals describes the electric separation of the DRL circuit from the circuit conditioning the electrical signals coming from the electrodes for the purpose of preventing the mentioned unwanted current injection in the medium, nor that of using individual reference voltages for each electrode for the purpose of comparing each of them with one of the signals supplied by each of the receiver electrodes in respective instrumentation or differential amplifiers.

### Description of the Invention

In view of the state of the art the main drawbacks of which have been set forth above, it is necessary to offer an alternative which enables solving said drawbacks, and which particularly prevents altering the balance in the potentials of the half-cells occurring in the conventional proposals upon injecting a current into a medium, generally a patient, through a reference electrode, such as that described above as a DRL electrode.

To that end the present invention relates in a first aspect to a weak electrical signal conditioning circuit, of the type comprising:
- at least one adjustable-gain instrumentation amplifier with a high input impedance, with:
- a first input in connection with a first receiver electrode in contact with a first area of a medium for receiving at least one of said weak electrical signals coming from said medium, and
- a second input in connection with a reference voltage,
- a reference voltage generating device in connection with a voltage supplying electrode in contact with a second area of said medium for applying a reference electrical signal to it so that the voltage existing in the medium has a value substantially equal to that of said electrical signal generated by said reference voltage generating device, and
- compensation means for compensating at least the direct current offsets experienced by at least said weak electrical signal received by said first receiver electrode.

Unlike the conventional proposals the circuit proposed by the first aspect of the invention is adapted for applying to the voltage supplying electrode, by means of said reference voltage generating device, a direct current electrical signal with a fixed value, the mentioned compensation means being insulated electrically with respect to the constant reference voltage generating device and substantially with respect to the voltage supplying electrode for assuring that there is no current flow through the medium as a result of the action of the compensation means, contrary to what occurred with the previously described conventional conditioning circuits, in which the fluctuations in the electrical signals provided by the receiver electrodes and the possible artifacts, or interfering signals, caused a current injection into the medium through the reference electrode, altering the balance in the potentials of the half-cells.

In the circuit proposed by the first aspect of the invention the mentioned compensation means are electrically independent from the reference voltage generating device, and comprise:
- a voltage generating device connected to the second input of the instrumentation amplifier, for generating and supplying to it a reference voltage electrical signal, for compensating at least the unwanted direct current offsets experienced by the weak electrical signal received by the first receiver electrode, and
- a control system in connection with said voltage generating device, and adapted for controlling it for the purpose of modifying the value of said reference voltage.

Said control system is generally connected with the output of said instrumentation amplifier for controlling the voltage generating device according to the output signal of the instrumentation amplifier.

For an embodiment the mentioned compensation means are adapted for, for the purpose of complementing said compensation carried out by means of applying said reference voltage, supplying the instrumentation amplifier, through a direct current offset adjustment input, with a direct current compensation adjustment signal for a direct current compensating at the output of the instrumentation amplifier representative of a variable voltage value determined according to the direct current offset to be compensated experienced by the weak electrical signal received by the first receiver electrode.

The compensation means are also adapted for compensating alternating interfering signals by means of supplying said electrical signal with a reference voltage and/or said adjustment signal, to said second input and to said direct current offset adjustment input of the instrumentation amplifier, respectively.

In the present application weak electrical signals are understood to be signals with low amplitude and/or coming from sources with high output impedances. The AC voltage values of such signals are typically less than 10 mV and can have DC components greater than the AC signal.

For a preferred embodiment the conditioning circuit proposed by the first aspect of the invention is applied to conditioning biopotential measurement signals on any part of the body of a patient, such as those obtained by means of electroencephalograms (EEG), electrocardiograms (ECG), electro-oculograms (EOG) or electromyograms (EMG), said medium being a patient, for the purpose of achieving a minimum current flow therethrough.

For said preferred embodiment the voltage supplying electrode is in contact with a contact area of said patient, forming with said constant voltage generating device a right leg circuit DRL for the purpose of achieving that the voltage of the patient is substantially equal to the voltage supplied by the constant voltage generating device.

A first aspect of the present invention relates to a method for controlling a weak electrical signal conditioning circuit, of the type which comprises:
- receiving at least one of said weak electrical signals coming from a medium, through a first receiver electrode in contact with a first area of said medium,
- sending said received weak electrical signal to a first input of an adjustable-gain instrumentation amplifier with a high input impedance,
- applying a reference voltage to a second input of said instrumentation amplifier,
- applying a reference electrical signal to a voltage supplying electrode in contact with a second area of said medium so that the voltage existing in the medium has a value substantially equal to that of said reference electrical signal applied to said voltage supplying electrode, and
- compensating at least the direct current offsets experienced by said weak electrical signal received by the first receiver electrode.

The method proposed by the first aspect of the invention is characterized in that:
- said reference electrical signal applied to the voltage supplying electrode is a direct current electrical signal with a fixed value, independent from the common-mode signal of said instrumentation amplifier,
- and in that said compensation of at least the direct current offsets experienced by said weak electrical signal received by the first receiver electrode, is automatically carried out by means of compensation means substantially electrically insulated with respect to said voltage supplying electrode for assuring that there is no current flow through the medium as a result of the action of the compensation means.

For an embodiment the method comprises carrying out said compensation of the direct current offsets experienced by said weak electrical signal received by the first receiver electrode by means of applying said reference voltage and modifying its value in a controlled manner.

The proposed method comprises carrying out said compensation or compensations for compensating the common-mode voltage variations of said instrumentation amplifier.

The method proposed by the first aspect of the invention is applied to conditioning biopotential measurement signals, said medium being a patient, for the purpose of achieving a minimum current flow through the medium.

Although it is not limited to it, the method proposed by the first aspect of the invention is applied to controlling a conditioning circuit according to the first aspect of the invention.

### Brief Description of the Drawings

The previous and other advantages and features will be more fully understood from the following detailed description of several embodiments with reference to the attached drawings, which must be considered in an illustrative and non-limiting manner, in which:
Figure 1 is a schematic depiction of the conditioning circuit proposed by the first aspect of the invention applied to conditioning biopotential signals of a patient, specifically ECG signals, for an embodiment for which the signals come from a single receiver electrode;
Figure 2 is a view similar to Figure 1, but for an embodiment for which the conditioning circuit is applied to conditioning signals coming from two receiver electrodes,
Figure 3 shows the method proposed by the second aspect of the invention, for an embodiment, by means of a flow chart.
Figures 4a to 4e are different views of a support supporting the circuit proposed by the first aspect of the invention and the electrodes connected thereto applied in the head of a patient, for the execution of an encephalogram, and
Figure 5 is a perspective view of a casing or case housing part of the conditioning circuit proposed by the first aspect of the invention.

### Detailed Description of Several Embodiments

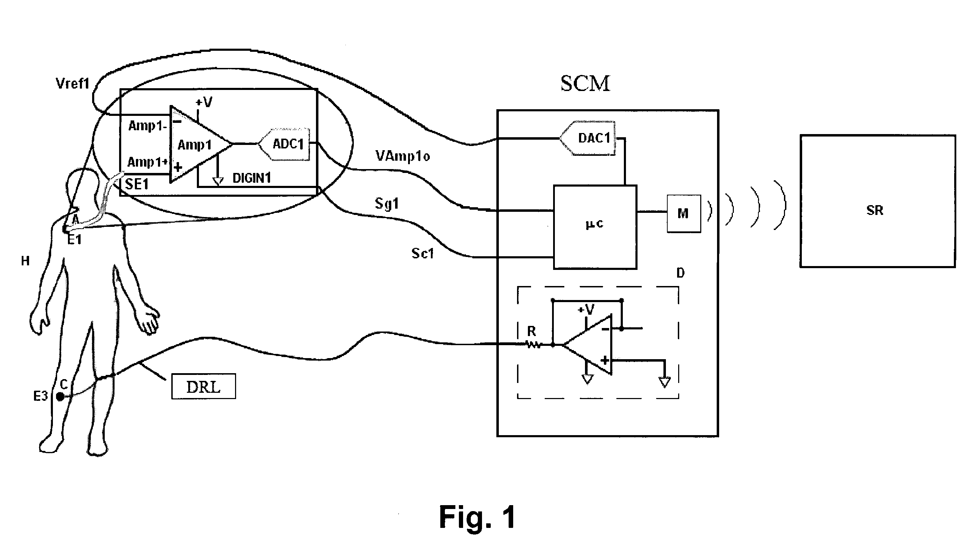

With reference to Figure 1, it shows the conditioning circuit proposed by the first aspect of the invention for an embodiment for which it is applied to conditioning biopotential signals representative of an ECG conducted on a patient H.

The circuit of Figure 1 comprises:
- an adjustable-gain instrumentation amplifier Amp1 with a high input impedance, with:
- a first input Amp1+ in connection with a first receiver electrode E1 in contact with a first area A of said patient H (area arranged in the upper right torso of the patient H) for receiving a weak electrical signal SE 1 there from, and
- a second input Amp1- in connection with a reference voltage Vref1,
- a reference voltage generating device D in connection with a voltage supplying electrode E3 in contact with a second area C of said patient H, in this case the right leg thereof, for applying a reference electrical signal to it so that the voltage existing in the medium, in this case a patient H, has a value substantially equal to that of said electrical signal generated by said reference voltage generating device D, which form a right leg circuit DRL with the electrode E3
- compensation means for compensating the direct current offsets experienced by said weak electrical signal SE1 received by the first receiver electrode E1.

As has been previously stated the circuit proposed by the first aspect of the invention is adapted for applying to the voltage supplying electrode E3, by means of the reference voltage generating device D, a DC electrical signal with a fixed value, said reference voltage generating device D being a constant voltage generating device D.

The constant voltage generating device D is formed, for the embodiments shown by Figures 1 and 2, by an optional amplifier with current limitation in order to comply with international recommendations, such that it is assured that the voltage supplied to the electrode E3 is very stable, said voltage being a precision voltage reference (precision band interval reference).

Since the compensation means are insulated electrically with respect to the constant voltage generating device D and substantially with respect to the voltage supplying electrode E3 (between E1 and E3 there is only the impedance of the patient H, which is very high) it is assured that there is no current flow through the medium H as a result of the action of the compensation means, contrary to conventional proposals in which said current flow causing changes in the potentials of the half-cells did occur.

The compensation means of the circuit proposed by the first aspect of the invention comprise, for the embodiment shown by Figure 1:
- a voltage generating device, which is a digital-to-analog converter DAC1 connected to the second input Amp1- of the instrumentation amplifier Amp1, for generating and supplying to it a reference voltage electrical signal Vref1 through said second input Amp1-, for the unwanted direct current offsets experienced by the weak electrical signal SE1 received by the first receiver electrode E1, and
- a control system in connection with said voltage generating device DAC1, and adapted for controlling it for the purpose of modifying the value of said reference voltage Vref1.

As has been previously stated, the mentioned compensation means are adapted for supplying the instrumentation amplifier Ampl1, through a direct current offset adjustment input DIGIN1, with a direct current compensation adjustment signal Sc1 for a direct current compensating at the output of the instrumentation amplifier Ampl1 representative of a variable voltage value determined according to the direct current offset to be compensated experienced by said weak electrical signal SE1 received by the first receiver electrode E1.

Moreover the compensation means of the circuit proposed by the first aspect of the invention are also adapted to compensate alternating interfering signals by means of supplying said reference voltage electrical signal Vref1 and/or said adjustment signal Sc1, according to the necessary compensation.

The compensation means are adapted for, according to the ratio between the output signal of the instrumentation amplifier Amp 1 and the dynamic range thereof:
- adjusting said gain of the instrumentation amplifier Amp1 by means of sending a gain adjustment signal Sg1 to a gain adjustment input DIGIN1 thereof, and/or
- carrying out said modification of the value of said reference voltage Vref1 to be applied to the second input Amp1- of the instrumentation amplifier Amp1, and/or
- modifying the value of said variable voltage, and therefore of said adjustment signal Sc1 representative thereof to be applied to said direct current offset adjustment input DIGIN1 of the instrumentation amplifier Ampl1.

For the embodiments shown by Figures 1 and 2, said gain adjustment input and said direct current offset adjustment input are one and the same input DIGIN1 for programming the instrumentation amplifier Amp1, said adjustment signals Sg1, Sc1 being digital signals.

The mentioned control system is formed by a microcontroller µc (or by a logic circuit for other embodiments not shown), forming part of a local electronic system SCM which also includes said voltage generating devices D, DAC1, said control system being connected to the output of the instrumentation amplifier Amp1 once digitized by an analog-to-digital converter ADC1, for monitoring it and operating accordingly, said operation including the mentioned control of the digital-to-analog converter DAC1 for modifying the value of the reference voltage Vref1.

Continuing with Figure 1, it also shows how the microcontroller µc comprises:
- an output connected to DAC 1 the output of which is connected to the second input Amp1- of the first instrumentation amplifier Amp1, for sending it the reference voltage electrical signal Vref1 after its conversion to an analog format, and
- another output connected to an adjustment input DIGIN1 of the instrumentation amplifier Amp1 for supplying it with said adjustment signal Sc1, Sc2, Sg1, Sg2 in digital format.

Figure 2 shows another embodiment similar to that shown by Figure 1 but in which the conditioning circuit is applied to conditioning biopotential signals coming from two electrodes E1 and E2.

In said circuit shown by Figure 2, it comprises a second adjustable-gain instrumentation amplifier Amp2 with a high input impedance, with:
- a first input Amp2+ in connection with a second receiver electrode E2 in contact with a third area B of the patient H (in this case an area arranged in the upper left torso of the patient H), for receiving another one of said weak electrical signals SE2, and
- a second input Amp2- in connection with a second reference voltage Vref2.

The compensation means illustrated in Figure 2 are also adapted for compensating the direct current offsets (and where appropriate alternating current offsets) experienced by said weak electrical signal SE2 received by the second receiver electrode E2, and comprise a second voltage generating device DAC2 connected to the second input Amp2- of the second instrumentation amplifier Amp2, for generating and supplying to it a second reference voltage electrical signal Vref2, for carrying out said compensation.

As is seen in Figure 2, the control system comprises the mentioned microcontroller µc also connected with said second voltage generating device DAC2, which is another digital-to-analog converter, and adapted for controlling it for the purpose of modifying the value of the second reference voltage Vref2 applied to the second input Amp2- of the second instrumentation amplifier Amp2.

The compensation means are adapted for compensating direct current offsets experienced by the weak electrical signal SE2 received by said second electrode E2 and alternating interfering signals, operating in a manner similar to how they operate with the differential amplifier Amp1, including the supply of a respective direct current compensation adjustment signal Sc2 and the sending of a gain adjustment signal Sg2 to an adjustment input DIGIN2 of the same Amp2.

The local control system SCM shown in Figure 2 includes both the microcontroller µc and the constant voltage generating device D, as well as the two digital-to-analog converters DAC 1 and DAC2.

The microcontroller µc of Figure 2 comprises:
- first and second outputs respectively connected to the first and second digital-to-analog converters DAC1, DAC2, and
- third and fourth outputs respectively connected to the adjustment inputs DIGIN1, DIGIN2 of the instrumentation amplifiers Amp1, Amp2, for supplying them with said adjustment signals Sc1, Sc2, Sg1, Sg2 in digital format.

The polarization voltages of the amplifiers Amp1, Amp2 and of the operational amplifier of the generating device D, and indicated as +V in Figures 1 and 2 come from batteries (not shown) included in the local electronic system SCM, such that some of the interferences caused by the mains voltage when said circuits are supplied by the mains supply are prevented.

A suitable instrumentation amplifier for being used as Amp1 and Amp2 is for example the programmable-gain instrumentation amplifier AD8555, although the circuit shown by Figures 1 and 2 is not limited to any specific instrumentation amplifier, provided that it is of the type which has a programmable gain and has one or more gain programming and direct current offset compensation inputs, such as the inputs DIGIN1 and DIGIN2 shown.

It is necessary to point out that the conditioning circuit of Figures 1 and 2 has been shown in a simplified and schematic manner, including the most important elements forming part thereof, but the inclusion of other elements (such as filters) that are common in this type of circuits (some of which have been mentioned in the "State of the Art" section) is also contemplated by the present invention.

In fact the instrumentation amplifiers Amp1, Amp2 themselves, such as the mentioned AD8555, include a circuit for suppressing radio frequency interferences with a low-pass filter with a bandwidth of a few kHz.

A low-pass (RC) filter (not shown) is used before the digitalization, which filter allows the signals within the passband of the filter to pass through while at the same time it limits the bandwidth of the signals which are outside the passband, thus reducing the possible noise in the input signals of the analog-to-digital converters ADC1, ADC2, and therefore obtaining digital signals VAmp1o, VAmp2o representative of interference-free analog signals.

It is necessary to point out that some of the components or elements of the local electronic system SCM have also not been shown so that Figures 1 and 2 are clearer when schematically illustrating the main acting components of the circuit proposed by the first aspect of the invention. One of said not shown elements is a low noise linear regulator (such as ADP3331) which reduces the noise of the analog output signals of the SCM, i.e., Vref1, Vref2, and the reference voltage of electrode E3 of the DRL circuit.

As is schematically shown in Figures 1 and 2, the local electronic system SCM comprises a communications module M adapted for wirelessly communicating with a remote control system SR, preferably in a two-way manner.

For an embodiment, said remote control system SR is adapted for wirelessly receiving, from the local electronic system SCM, the digital values representative of the output signal VAmp1o, VAmp2o of the instrumentation amplifier (Figure 1) or amplifiers (Figure 2) Amp1, Amp2, and for analyzing them.

For an embodiment, the remote control system SR is adapted for carrying out, automatically or if necessary with the intervention of an operator (for example for choosing a control program to be applied), at least part of the gain adjustments of the instrumentation amplifiers Amp1, Amp2, and/or the modification of the values of the reference voltages Vref1, Vref2, and/or the modification of the adjustment signals Sc1, Sc2, and for carrying out the corresponding sendings of the digital values of the adjustment signals Sc1, Sc2, Sg1, Sg2 and/or of reference voltages Vref1, Vref2 to the local electronic system SCM.

For another embodiment, the local electronic system SCM is adapted for carrying out the gain adjustments of the instrumentation amplifiers Amp1, Amp2, and/or the modification of the value of the reference voltages Vref1, Vref2 and/or the modification of the adjustment signals Sc1, Sc2.

Such remote control system SR is, for an embodiment, a computerized system in connection with display means, such as a screen, for displaying the output signals VAmp1o, VAmp2o, i.e., for the case of an ECG, the signals representative thereof.

The mentioned remote computerized system SR preferably comprises a series of both input and output peripherals in order to enable its use by an operator for example for the mentioned embodiment in which part of the adjustments are carried out by the remote system SR.

The remote system SR obviously also has a communications module (not shown) which is internal or external (for example connected to a USB port) and which can wirelessly communicate with the communications module M of the local electronic system SCM with the same technology and protocols (for example Zigbee).

The main objective of using wireless communication is eliminating possible signal interferences with the mains frequency (50 or 60 Hz) caused by the use of cables. This is so due to the fact that said wireless interface or communication eliminates the main stray capacitance between the body of the patient and ground which occurred in the mentioned cables of conventional proposals. Said wireless communication obviously also provides great autonomy to the patient which the wiring does not allow.

For an embodiment, the instrumentation amplifiers Amp1, Amp2, associated circuitry, and in general the local electronic system SCM are supported by a support C which also supports all or part of the electrodes E1, E2, E3, together with the instrumentation amplifiers Amp1, Amp2 and associated circuitry ADC1, ADC2, filters (not shown), etc.

The fact that the signals of the receiver electrodes E1, E2 are amplified and digitized in situ, i.e., with the instrumentation amplifiers Amp1, Amp2, and other associated circuitry, arranged very close to the electrodes, considerably eliminates the interfering noise which conventionally occurs when the active electrodes are far from the amplifying stages, due to the high degree of rejection to the common-mode of the instrumentation amplifiers it eliminates the mains frequency noise common to the two electrodes E1, E2, and to the absence of cables between the electrodes E1, E2 and the amplification electronics. After the digitization in situ the digitalized output signal VAmp1o, VAmp2o (see Figures 1 and 2) has no interference problems.

The reference voltage of the supplying electrode E3 of the DRL circuit has a high immunity to noise, due to the fact that it comes from a dedicated noise cancellation circuit, which in Figures 1 and 2 is formed by a voltage follower based on an operational amplifier.

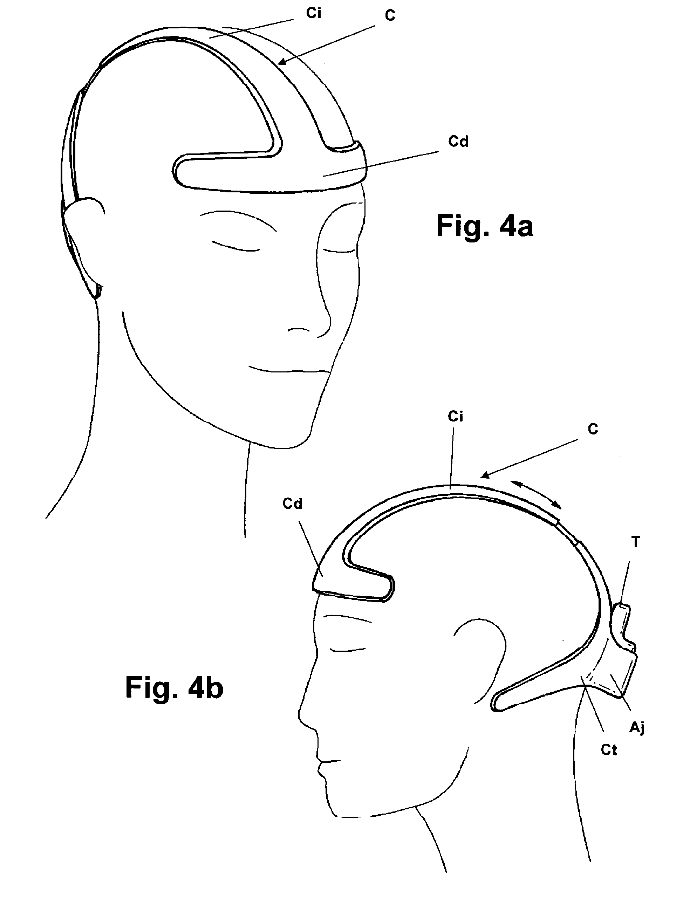

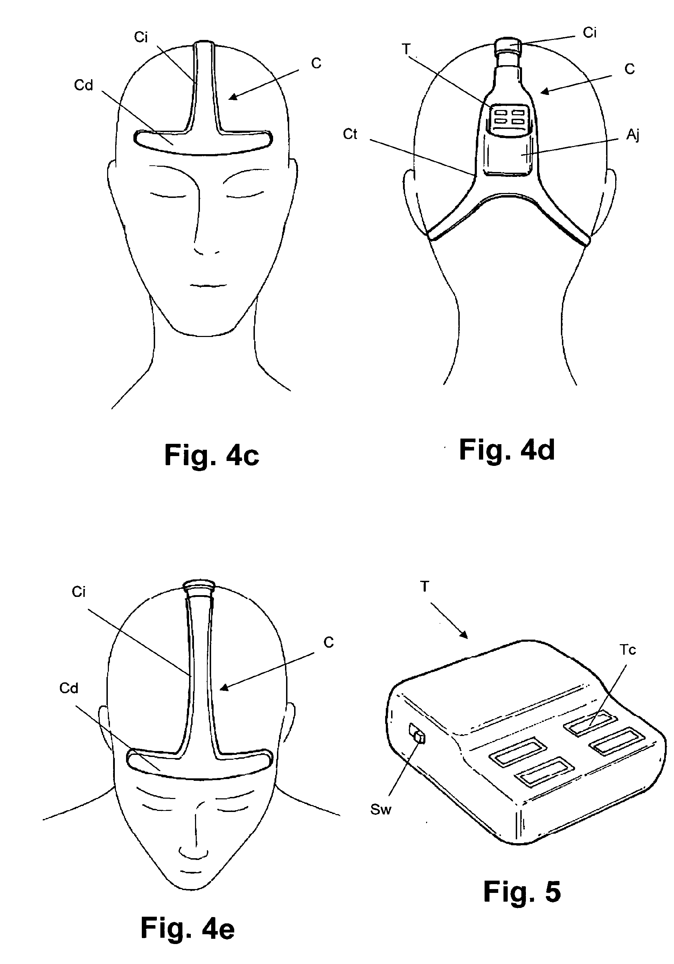

Figures 4a to 4e show a case for which (unlike that shown by Figures 1 and 2 referring to an ECG system) the biopotential signals to be conditioned are brain signals, for the execution of an encephalogram, in which the mentioned support C is coupled in the head of the patient.

For the embodiment shown in said Figures 4a to 4e the electrodes used are integrated in a front portion Cd of the support C, such that they are in contact with the forehead of the patient, although any other location which is considered suitable for performing the ECG is also possible.

The intermediate portion Ci of the support C forms a strip Ci running along the head connecting the front portion Cd and a back portion Ct of the support C. Said strip Ci can be extended for the purpose of adapting the support C to different head sizes.

There is a housing Aj defined in the back portion Ct of the support C for the local electronic system SCM, which is in turn housed inside a case T, which is shown with greater detail in Figure 5.

In the case T shown in said Figure 5, a series of connectors Tc is observed for connecting the ends of corresponding cables (not shown) connected to the instrumentation amplifiers of the receiver electrodes, and to the voltage supplying electrode, and are running along the strip Ci of the support C and are integrated therein.

Said case T also has on one of its sides a switch Sw for its handling by an operator for the purpose of activating/deactivating the local electronic system (SCM).

As has been indicated in a previous section, the present invention also relates, in a second aspect, to a method for controlling a weak electrical signal conditioning circuit which, although it is not limited to it, is applied to controlling a conditioning circuit according to the first aspect of the invention.

For the embodiments for which the method is applied to the proposed circuit according to the first aspect of the invention, specifically for the embodiments shown by Figures 1 and 2, the method comprises carrying out the actions already described in the description of the first aspect of the invention, including the generation, modification, adjustment and supply of the different signals Vref1, Vref2, Sc1, Sc2, Sg1 and Sg2, and monitoring the output signals VAmp1p, VAmp2p of the amplifiers Amp1, Amp2.

As has been previously described with reference to the circuit proposed by the first aspect of the invention, the method proposed by the second aspect also comprises carrying out said compensations performing the mentioned actions for generating, modifying, adjusting and supplying signals Vref1, Vref2, Sc1, Sc2, Sg1 and Sg2, according to the ratio between the output signal of the instrumentation amplifier or amplifiers Amp1, Amp2 and the dynamic range thereof.

An embodiment of the method proposed by the second aspect of the invention is described below with reference to an instrumentation amplifier Amp1, although the second Amp2 or even other additional amplifiers are controlled in the same way as that explained below for Amp1.

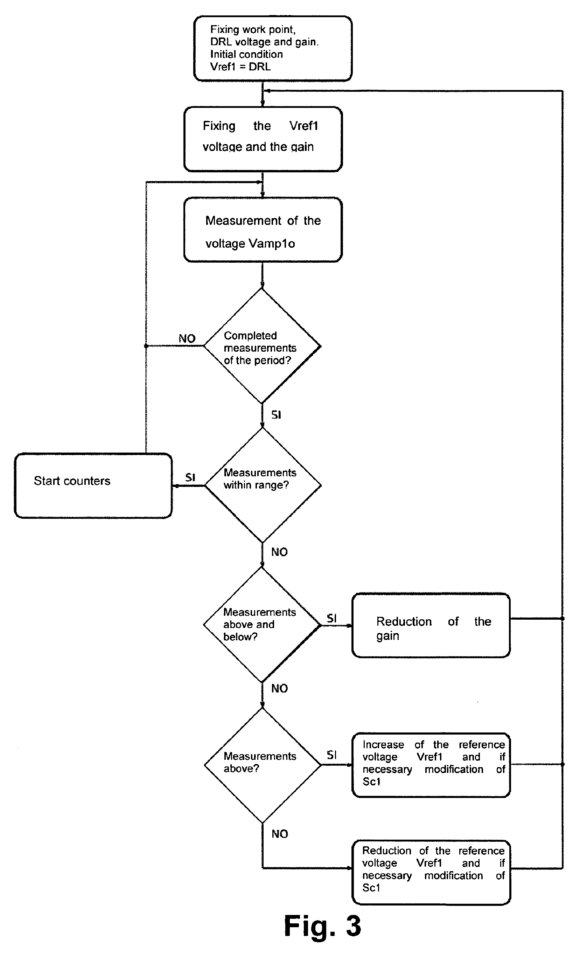

For said embodiment, shown by means of a flow chart in Figure 3, the method proposed by the second aspect comprises carrying out the following steps, taking into account said ratio between the output signal of the instrumentation amplifier Amp1 and the dynamic range thereof:
a) fixing an initial work point which includes predetermining a value for the gain adjustment signal Sg1, according to the desired gain, and a substantially equal value for the reference electrical signal to be applied to the voltage supplying electrode E3 and for the reference voltage Vref1, i.e., equal to that generated by the constant voltage generating device D (see Figures 1 and 2).
   Said step a) is shown in the first and second boxes (counting from the top) of the flow chart of Figure 3.
b) monitoring the digitized output of the instrumentation amplifier Amp1 for a predetermined number of samples or period;
   Step b) is shown in the third and fourth boxes of the flow chart of Figure 3 (in which the monitored values are indicated as "measurements"). Said third and fourth boxes are connected by a return path (from the fourth to the third box) indicating that if the mentioned number of samples has not been reached, samples must continue to be acquired until it is so, and then the next box is entered into, i.e., the next step is carried out:
c) checking if the values of the signal VAmp1o obtained in said monitoring of said step b) are within the dynamic range of the instrumentation amplifier Amp1 (fifth box counting from the top), and:
   - if as a result of said step c) it is determined that the values of the monitored signal VAmp1o are within the dynamic range of the instrumentation amplifier Amp1, starting a series of counters regarding at least said number of samples or period and said monitored signal VAmp1o, and carrying out said steps b) and c) again;
   - if as a result of said step c) it is determined that the values of the monitored signal VAmp1o are outside the dynamic range of the instrumentation amplifier Amp1, passing to the sixth box counting from the top which refers to a disjunctive, according to the response of which the method comprises alternatively carrying out the following steps:
d1) if there are values of the monitored signal VAmp1o above and below the dynamic range of the instrumentation amplifier Amp1, reducing the gain thereof by means of modifying said gain adjustment signal Sg1 (box to the right of the sixth box counting from the top) and its corresponding application (second box); and carrying out said steps b) and c) again;
d2) if there are only values of the monitored signal VAmp1o above the dynamic range of the instrumentation amplifier Amp1 (affirmative response to the disjunctive of the seventh box counting from the top), at least increasing the value of the reference voltage Vref1 (box to the right of the seventh box counting from the top), applying it (second box), and carrying out said steps b) and c) again; or
d3) if there are only values of the monitored signal VAmp1o below the dynamic range of the instrumentation amplifier Amp1 (negative response to the disjunctive of the seventh box counting from the top), at least decreasing the value of the reference voltage Vref1 (last box of the flow chart of Figure 3 counting from the top), applying it (second box), and carrying out said steps b) and c) again.

As is indicated in the flow chart of Figure 3, if it is necessary:
said step d2) further comprises modifying the direct current offset adjustment signal Sc1 and applying it to the direct current offset adjustment input DIGIN1 of the instrumentation amplifier Ampl1, in order to reduce the direct current level in the monitored signal VAmp1o; and
said step d3) further comprises modifying the direct current offset adjustment signal Sc1 and applying it to the direct current offset adjustment input DIGIN1 of the instrumentation amplifier Ampl1, in order to increase the direct current level in the monitored signal VAmp1o.

As has been previously mentioned the method proposed by the second aspect of the invention comprises compensating the direct current offsets experienced by other weak electrical signal or signals received by other receiver electrodes in contact with other areas of the patient H, in an analogous manner to how the compensation with the weak electrical signal SE1 received by the first receiver electrode E1 is carried out.

A person skilled in the art could introduce changes and modifications in the embodiments described without departing from the scope of the invention as it is defined in the attached claims.

## Claims

1. A weak electrical signal conditioning circuit, of the type comprising:
- at least one adjustable-gain instrumentation amplifier (Amp1) with a high input impedance, with:
- a first input (Amp1+) in connection with a first receiver electrode (E1) in contact with a first area (A) of a medium (H) for receiving at least one weak electrical signal (SE1) coming from said medium (H), and
- a second input (Amp1-) in connection with a reference voltage (Vref1),
- a reference voltage generating device (D) in connection with a voltage supplying electrode (E3) in contact with a second area (C) of said medium (H) for applying a reference electrical signal to said electrode (E3) so that the voltage existing in the medium (H) has a value substantially equal to that of said electrical signal generated by said reference voltage generating device (D),
- compensation means for compensating at least the direct current offsets experienced by at least said weak electrical signal (SE1) received by the first receiver electrode (E1),
said circuit being **characterized in that** said reference voltage generating device (D) is a constant voltage generating device (D), adapted for applying to the voltage supplying electrode (E3) said reference electrical signal in the form of a direct current electrical signal with a fixed value, and **in that** said compensation means comprise at least:
- a voltage generating device (DAC1) connected to said second input (Amp1-) of said at least one instrumentation amplifier (Amp1), for generating and supplying to it a reference voltage electrical signal (Vref1) through said second input (Amp1-), for compensating at least the unwanted direct current offsets experienced by the weak electrical signal (SE1) received by the first receiver electrode (E1), and
- a control system in connection with said voltage generating device (DAC1) and adapted for controlling it for the purpose of modifying the value of said reference voltage (Vref1),
said compensation means being insulated electrically with respect to said constant voltage generating device (D) and substantially with respect to said voltage supplying electrode (E3) for assuring that there is no current flow through the medium (H) as a result of the action of the compensation means.

2. The circuit according to claim 1, **characterized in that** said compensation means are adapted for, for the purpose of complementing said compensation carried out by means of applying said reference voltage (Vref1), supplying the instrumentation amplifier (Ampl1), through a direct current offset adjustment input (DIGIN1), with a direct current compensation adjustment signal (Sc1) for a direct current compensating at the output of the instrumentation amplifier (Ampl1) representative of a variable voltage value determined according to the direct current offset to be compensated experienced by at least said weak electrical signal (SE1) received by the first receiver electrode (E1).

3. The circuit according to claim 1 or 2, **characterized in that** said compensation means are also adapted for compensating alternating interfering signals by means of supplying said electrical signal with a reference voltage (Vref1) and/or said adjustment signal (Sc1), to said second input (Amp1-) and to said direct current offset adjustment input (DIGIN1) of the instrumentation amplifier (Amp1), respectively.

4. The circuit according to claim 1, 2 or 3, **characterized in that** the compensation means are adapted for, according to at least the ratio between the output signal of the instrumentation amplifier (Amp1) and the dynamic range thereof:
- adjusting said gain of the instrumentation amplifier (Amp1) by means of sending a gain adjustment signal (Sg1) to a gain adjustment input (DIGIN1) thereof, and/or
- carrying out said modification of the value of said reference voltage (Vref1) to be applied to the second input (Amp1-) of the instrumentation amplifier (Amp1), and/or
- modifying the value of said variable voltage, and therefore said adjustment signal (Sc1) representative thereof to be applied to said direct current offset adjustment input (DIGIN1) of the instrumentation amplifier (Ampl1).

5. The circuit according to claim 4, **characterized in that** said gain adjustment input and said direct current offset adjustment input are one and the same input (DIGIN1) for programming the instrumentation amplifier (Amp1), said adjustment signals (Sg1, Sc1) being digital signals.

6. The circuit according to any of the previous claims, **characterized in that** it is applied to conditioning biopotential measurement signals, said medium (H) being a patient (H), for the purpose of achieving a minimum current flow through the medium (H).

7. The circuit according to claim 6, **characterized in that** said voltage supplying electrode (E3) is in contact with a contact area (C) of said patient (H), forming with said constant voltage generating device (D) a right leg circuit (DRL) for the purpose of achieving that the voltage of the medium (H) is substantially equal to the voltage supplied by the constant voltage generating device (D).

8. The circuit according to any of the previous claims, **characterized in that** it comprises at least one second adjustable-gain instrumentation amplifier (Amp2) with a high input impedance, with:
- a first input (Amp2+) in connection with a second receiver electrode (E2) in contact with a third area (B) of said medium (H) for receiving at least another one of said weak electrical signals (SE2) coming from the medium (H), and
- a second input (Amp2-) in connection with a second reference voltage (Vref2), said compensation means being adapted for also compensating at least the direct current offsets experienced by said weak electrical signal (SE2) received by said second electrode (E2),
**in that** said compensation means comprise a second voltage generating device (DAC2) connected to said second input (Amp2-) of said second instrumentation amplifier (Amp2), for generating and supplying to it second reference voltage electrical signal (Vref2) through said second input (Amp2-), for compensating at least the unwanted direct current offsets experienced by the weak electrical signal (SE2) received by the second receiver electrode (E2),
and **in that** said control system is also connected with said second voltage generating device (DAC2), and adapted for controlling it for the purpose of modifying the value of said second reference voltage (Vref2) applied to the second input (Amp2-) of the second instrumentation amplifier (Amp2).

9. The circuit according to claim 8, **characterized in that** said compensation means are adapted for compensating direct current offsets experienced by the weak electrical signal (SE2) received by said second electrode (E2) and alternating interfering signals, operating in a manner analogous to how they operate with the instrumentation amplifier (Amp1), including the supply of a respective direct current compensation adjustment signal (Sc2) and the sending of a gain adjustment signal (Sg2) to an adjustment input (DIGIN2) of same (Amp2).

10. The circuit according to any of claims 1 to 9, **characterized in that** it comprises a local electronic system (SCM) including said control system, which is formed by at least one microcontroller (µc) or logic circuit, and said voltage generating devices (D, DAC1, DAC2), said control system being connected to the output of the instrumentation amplifier (Amp1), or outputs (Amplo, Amp2o) of the instrumentation amplifiers (Amp1, Amp2) when is the case, for monitoring them and operating accordingly.

11. The circuit according to claim 10, **characterized in that** said microcontroller (µc) or logic circuit comprises:
- first and second outputs respectively connected to said first and second voltage generating devices (DAC1, DAC2), which are respective digital-to-analog converters, the outputs of which are respectively connected to the second input (Amp1-) of the first instrumentation amplifier (Amp1) and to the second input (Amp2-) of the second instrumentation amplifier (Amp2), for sending to them said reference voltage electrical signals (Vref1, Vref2) after their conversion to an analog format, and
- third and fourth outputs respectively connected to said adjustment inputs (DIGIN1, DIGIN2) of the instrumentation amplifiers (Amp1, Amp2), for supplying them with said adjustment signals (Sc1, Sc2, Sg1, Sg2) in digital format.

12. The circuit according to claim 10 or 11, **characterized in that** at least said instrumentation amplifier (Amp1), or said instrumentation amplifiers (Amp1, Amp2) when is the case, and associated circuitry are supported by a support (C) which also supports at least one of said electrodes (E1, E2, E3).

13. The circuit according to claim 12, **characterized in that** at least part of said local electronic system (SCM) is also supported by said support (C).

14. The circuit according to claim 13, **characterized in that** said local electronic system (SCM) comprises a communications module (M) adapted for wirelessly communicating with a remote control system (SR).

15. The circuit according to claim 14, **characterized in that** said communications module (M) is adapted for communicating with said remote control system (SR) in a two-way manner.

16. The circuit according to claim 14 or 15, **characterized in that** said remote control system (SR) is adapted for wirelessly receiving, from the local electronic system (SCM), the digital values representative of the output signal (VAmp1o, VAmp2o) of the instrumentation amplifier or amplifiers (Amp1, Amp2), and for analyzing them.

17. The circuit according to claim 14, 15 or 16, **characterized in that** said remote control system (SR) is adapted for carrying out at least part of:
- said adjustment of said gain of the instrumentation amplifier or amplifiers (Amp1, Amp2), and/or
- said modification of the value of said reference voltage or voltages (Vref1, Vref2) to be applied to the second input (Amp1-, Amp2-) of the instrumentation amplifier or amplifiers (Amp1, Amp2), and/or
- said modification of said adjustment signal or signals (Sc1, Sc2) to be applied to said direct current offset adjustment input (DIGIN1, DIGIN2) of the instrumentation amplifier or amplifiers (Ampl1, Amp2),
and for carrying out the corresponding sendings of the digital values of adjustment signals (Sc1, Sc2, Sg1, Sg2) and/or of reference voltages (Vref1, Vref2) to the local electronic system (SCM).

18. The circuit according to any of the claims 11 to 15, **characterized in that** said local electronic system (SCM) is adapted for carrying out:
- said adjustment of said gain of the instrumentation amplifier or amplifiers (Amp1, Amp2), and/or
- said modification of the value of said reference voltage or voltages (Vref1, Vref2) to be applied to the second input (Amp1-, Amp2-) of the instrumentation amplifier or amplifiers (Amp1, Amp2), and/or
- said modification of said adjustment signal or signals (Sc1, Sc2) to be applied to said direct current offset adjustment inputs (DIGIN1, DIGIN2) of the instrumentation amplifier or amplifiers (Ampl1, Amp2).

19. A method for controlling a weak electrical signal conditioning circuit, of the type which comprises:
- receiving at least one of said weak electrical signals (SE1) coming from a medium (H), through a first receiver electrode (E1) in contact with a first area (A) of said medium (H),
- sending said received weak electrical signal (SE1) to a first input (Amp1+) of an adjustable-gain instrumentation amplifier (Amp1) with a high input impedance,
- applying a reference voltage (Vref1) to a second input (Amp1-) of said instrumentation amplifier (Amp1),
- applying a reference electrical signal to a voltage supplying electrode (E3) in contact with a second area (C) of said medium (H) so that the voltage existing in the medium (H) has a value substantially equal to that of said reference electrical signal applied to said voltage supplying electrode (E3),
- compensating at least the direct current offsets experienced by at least said weak electrical signal (SE1) received by the first receiver electrode (E1),
said method being **characterized in that**:
- said reference electrical signal applied to the voltage supplying electrode (E3) is a direct current electrical signal with a fixed value, independent from the common-mode signal of said instrumentation amplifier (Amp1),
- and **in that** said compensation of at least the direct current offsets experienced by at least said weak electrical signal (SE1) received by the first receiver electrode (E1), is automatically carried out by means of compensation means substantially electrically insulated with respect to said voltage supplying electrode (E3) for assuring that there is no current flow through the medium (H) as a result of the action of the compensation means.

20. The method according to claim 19, **characterized in that** it comprises carrying out said compensation of at least the direct current offsets experienced by at least said weak electrical signal (SE1) received by the first receiver electrode (E2), by means of applying said reference voltage (Vref1) and modifying its value in a controlled manner.

21. The method according to claim 20, **characterized in that** it comprises complementing said compensation carried out by means of applying said reference voltage (Vref1), by means of supplying the instrumentation amplifier (Ampl1), through a direct current offset adjustment input (DIGIN1), with a direct current compensation adjustment signal (Sc1) for a direct current compensating at the output of the instrumentation amplifier (Ampl1) representative of a variable voltage value determined according to the direct current offset to be compensated experienced by at least said weak electrical signal (SE1) received by the first receiver electrode (E1).

22. The method according to claim 19, 20 or 21, **characterized in that** it comprises also compensating alternating interfering signals by means of supplying said reference voltage electrical signal (Vref1) and/or said adjustment signal (Sc1), to said second input (Amp1-) and to said direct current offset adjustment input (DIGIN1) of the instrumentation amplifier (Amp1), respectively.

23. The method according to any of the claims 19 to 21, **characterized in that** it comprises carrying out said compensation or compensations for compensating the common-mode voltage variations of at least said instrumentation amplifier (Amp1).

24. The method according to any of the claims 19 to 23, **characterized in that** it comprises carrying out said compensation or compensations according to at least the ratio between the output signal of the instrumentation amplifier (Amp1) and the dynamic range thereof, by means of carrying out at least one of the following actions, or a combination thereof:
- adjusting said gain of the instrumentation amplifier (Amp1) by means of sending a gain adjustment signal (Sg1) to a gain adjustment input (DIGIN1) thereof;
- carrying out said modification of the value of said reference voltage (Vref1) to be applied to the second input (Amp1-) of the instrumentation amplifier (Amp1);
- modifying the value of said variable voltage, and therefore said adjustment signal (Sc1) representative thereof to be applied to said direct current offset adjustment input (DIGIN1) of the instrumentation amplifier (Ampl1).

25. The method according to claim 24 when depending on claim 21 or on claim 22, **characterized in that** it comprises carrying out the following steps:
a) fixing an initial work point which includes predetermining a value for said gain adjustment signal (Sg1), according to the desired gain, and a substantially equal value for said reference electrical signal to be applied to the voltage supplying electrode (E3) and for said reference voltage (Vref1) to be applied to the second input (Amp1-) of the instrumentation amplifier (Amp1);
b) monitoring the output of the instrumentation amplifier (Amp1) for a predetermined number of samples or period;
c) checking if the values of the signal (VAmp1o) obtained in said monitoring of said step b) are within the dynamic range of the instrumentation amplifier (Amp1), and:
- if as a result of said step c) it is determined that the values of the monitored signal (VAmp1o) are within the dynamic range of the instrumentation amplifier (Amp1), starting a series of counters relative to at least said number of samples or period and said monitored signal (VAmp1o), and carrying out said steps b) and c) again;
- if as a result of said step c) it is determined that the values of the monitored signal (VAmp1o) are outside the dynamic range of the instrumentation amplifier (Amp1), alternatively carrying out the following steps:
d1) if there are values of the monitored signal (VAmp1o) above and below the dynamic range of the instrumentation amplifier (Amp1), reducing the gain thereof by means of modifying and applying said gain adjustment signal (Sg1); and carrying out said steps b) and c) again;
d2) if there are only values of the monitored signal (VAmp1o) above the dynamic range of the instrumentation amplifier (Amp1), at least increasing the value of said reference voltage (Vref1) to be applied to the second input (Amp1-) of the instrumentation amplifier (Amp1), applying it, and carrying out said steps b) and c) again; or
d3) if there are only values of the monitored signal (VAmp1o) below the dynamic range of the instrumentation amplifier (Amp1), at least decreasing the value of said reference voltage (Vref1) to be applied to the second input (Amp1-) of the instrumentation amplifier (Amp1), applying it, and carrying out said steps b) and c) again.

26. The method according to claim 25, **characterized in that**:
said step d2) further comprises modifying said direct current offset adjustment signal (Sc1) and applying it to said direct current offset adjustment input (DIGIN1) of the instrumentation amplifier (Ampl1), in order to reduce the direct current level in the monitored signal (VAmp1o); and
said step d3) further comprises modifying said direct current offset adjustment signal (Sc1) and applying it to said direct current offset adjustment input (DIGIN1) of the instrumentation amplifier (Ampl1), in order to increase the direct current level in the monitored signal (VAmp1o).

27. The method according to any of the claims 19 to 26, **characterized in that** it comprises compensating at least the direct current offsets experienced by another weak electrical signal (SE2) received by a second receiver electrode (E2) in contact with a third area (B) of said medium (H), in a manner analogous to how the compensation is carried out with said weak electrical signal (SE1) received by said first receiver electrode (E1).

28. The method according to any of the claims 19 to 27, **characterized in that** it is applied to conditioning biopotential measurement signals, said medium (H) being a patient (H), for the purpose of achieving a minimum current flow through the medium (H).

29. The method according to any of the claims 19 a 28, **characterized in that** it is applied to controlling a conditioning circuit as per any of the claims 1 to 18.
